# EUROPÄISCHE PATENTSCHRIFT

(11) **EP 1 141 273 B1**
(45) Veröffentlichungstag und Bekanntmachung des Hinweises auf die Patenterteilung: **01.07.2009**
(21) Anmeldenummer: 00901066.1
(22) Anmeldetag: 05.01.2000
(51) Int. Cl.: C12N 15/10, C12Q 1/68

(54) **METHODE ZUR ISOLIERUNG VON DNA AUS BIOLOGISCHEN MATERIALIEN**
METHOD FOR ISOLATING DNA FROM BIOLOGICAL MATERIALS
PROCEDE POUR ISOLER DE L'ADN CONTENU DANS DES MATIERES BIOLOGIQUES

(30) Priorität: 11.01.1999 DE 19900638
(43) Veröffentlichungstag der Anmeldung: 10.10.2001
(60) Teilanmeldung: 08014519.6
(73) Patentinhaber: Max-Planck-Gesellschaft zur Förderung der Wissenschaften e.V., 80539 München (DE); QIAGEN GmbH, 40724 Hilden (DE)
(72) Erfinder: MÜLLER, Oliver, D-44236 Dortmund (DE); SPRENGER-HAUSSELS, Markus, D-42697 Solingen (DE); BASTIAN, Helge, D-40597 Düsseldorf-Benrath (DE); VOLLERT, Stefanie, D-65719 Hofheim-Lorsbach (DE)
(74) Vertreter: Weiss, Wolfgang
(86) Internationale Anmeldenummer: PCT/EP2000/000052
(87) Internationale Veröffentlichungsnummer: WO 2000/042177

(56) Entgegenhaltungen:
- WO-A-97/07239
- US-A- 5 817 798
- GOUVEA V ET AL: "Identification of hepatitis E virus in clinical specimens: amplification of hydroxyapatite-purified virus RNA and restriction endonuclease analysis." JOURNAL OF VIROLOGICAL METHODS, (1997 DEC) 69 (1-2) 53-61. , XP000901005
- FLAGSTAD O ET AL: "Reliable noninvasive genotyping based on excremental PCR of nuclear DNA purified with a magnetic bead protocol." MOLECULAR ECOLOGY MAY, 1999, Bd. 8, Nr. 5, Mai 1999 (1999-05), Seiten 879-883, XP000901057 ISSN: 0962-1083
- BRETAGNE S ET AL: "Detection of Echinococcus multilocularis DNA in fox faeces using DNA amplification." PARASITOLOGY 1993, Bd. 106, Nr. 2, 1993, Seiten 193-199, XP000901017 ISSN: 0031-1820
- DEUTER R ET AL: "A METHOD FOR PREPARATION OF FECAL DNA SUITABLE FOR PCR" NUCLEIC ACIDS RESEARCH,GB,OXFORD UNIVERSITY PRESS, SURREY, Bd. 23, Nr. 18, 25. September 1995 (1995-09-25), Seiten 3800-3801, XP002017414 ISSN: 0305-1048

## Beschreibung

Die Erfindung betrifft ein Verfahren zur Stabilisierung, Reinigung oder/und Isolierung von Nukleinsäuren aus biologischen Materialien, insbesondere Stuhlproben, die Verunreinigungen und Inhibitoren bzw. Störsubstanzen enthalten können. Weiterhin wird ein für die Durchführung des erfindungsgemäßen Verfahrens geeigneter Reagenzienkit beschrieben.

Zahlreiche Beispiele aus verschiedenen Forschungsbereichen belegen die Bedeutung der Analyse von Nukleinsäuren aus biologischen Materialien, die mit Substanzen verunreinigt sind, welche Nukleinsäuren während der Lagerung schädigen und eine enzymatische Manipulation der Nukleinsäuren, z.B. durch Amplifikation hemmen. Daher ist es für die Brauchbarkeit der in den biologischen Materialien enthaltenen Nukleinsäuren für weitere Analysen wichtig, daß diese Substanzen nur in sehr niedrigen Konzentrationen vorhanden sind oder gänzlich aus der Probe entfernt werden.

Eine besondere Bedeutung besitzt die Analyse von Nukleinsäuren aus fäkalen Proben. Eine wichtige medizinische Anwendung ist der Nachweis tumorspezifischer Veränderungen von nukleärer Human-DNA aus Stuhl, die als Parameter bei der Frühdiagnose von Tumoren des Verdauungstrakts dienen können. Ebenso gewinnt der Nachweis bakterieller und viraler Infektionserreger aus Stuhlproben durch auf Nukleinsäuren basierende Testverfahren zunehmend an Bedeutung.

Für die Aufreinigung von Nukleinsäuren aus Stuhlproben ist die Anwendung einer Kombination verschiedener Reinigungsschritte wie Proteasebehandlung, Phenol/Chloroform-Extraktion, Bindung von Nukleinsäuren an Silika in Anwesenheit chaotroper Salze, Gelfiltration, Anionenaustauschchromatographie sowie der Einsatz kationischer Detergenzien bekannt. Die mit diesen Verfahren aus Stuhlproben isolierten Nukleinsäuren sind jedoch im allgemeinen instabil und verhalten sich oftmals problematisch in nachfolgenden enzymatischen Reaktionen wie z.B. PCR. Ursache hierfür sind Substanzen, die zusammen mit der Nukleinsäure isoliert werden und diese schädigen sowie enzymatische Reaktionen inhibieren. Im Stuhl enthaltene Inhibitorklassen sind - soweit bekannt - Hämoglobin und dessen Metaboliten, Gallensäuren und Gallensäurederivate sowie Polysaccharide.

In PCT/EP/96/03595 wird ein Verfahren zur Reinigung, Stabilisierung oder/und Isolierung von Nukleinsäuren aus biologischen Materialien, insbesondere Fäkalien, beschrieben, bei dem man einer Nukleinsäuren enthaltenden Probe aus biologischen Materialien eine Adsorptionsmatrix zur Bindung von Verunreinigungen zusetzt. Vorzugsweise verwendet man eine Adsorptionsmatrix auf Kohlenhydratbasis, z.B. Stärke, Cellulose, Glycogen oder/und andere biogene oder nicht biogene Kohlenhydrate oder Mischungen davon, wobei Mehle aus Getreide, Erbsen, Mais, Kartoffeln oder Bestandteile daraus oder Mischungen bevorzugt sind. Als besonders geeignet zur Aufreinigung von Nukleinsäuren aus Stuhlproben haben sich Mischungen aus gereinigten Kohlenhydraten oder/und Mehlen, insbesondere Mischungen aus Cellulose und Kartoffelmehl erwiesen.

Mit dem in PCT/EP96/03595 beschriebenen Verfahren werden in manchen Fällen jedoch die Nukleinsäuren schädigenden Substanzen und PCR-Inhibitoren nicht vollständig entfernt. Bei einem - variablen - Anteil von inhibitorischen Stuhlproben ist nach Aufreinigung unter Verwendung des Standardprotokolls die anschließende enzymatische Behandlung der Nukleinsäuren nicht möglich.

Eine Aufgabe der vorliegenden Erfindung war somit die Bereitstellung eines Verfahrens zur Aufreinigung von Nukleinsäuren, das die Nachteile des Standes der Technik mindestens teilweise beseitigt und das insbesondere eine reproduzierbare Aufreinigung von Nukleinsäuren aus "inhibitorischen Proben" ermöglicht.

Überraschenderweise wurde festgestellt, daß die Aufreinigung von Nukleinsäuren auch aus inhibitorischen Proben verbessert werden kann, wenn eine oder mehrere der im folgenden genannten Maßnahmen ergriffen werden:
(a) Verwendung eines Extraktionspuffers mit einem sauren bis neutralen pH-Wert,
(b) Verwendung eines Extraktionspuffers mit einem hohen Salzgehalt und
(c) Verwendung eines Extraktionspuffers, der eine phenolneutralisierende Substanz enthält.

Ein Gegenstand der Erfindung ist somit ein Verfahren zur Reinigung, Stabilisierung oder/und Isolierung von Nukleinsäuren aus biologischen Materialien, wobei man der Nukleinsäuren enthaltenden Probe einen Extraktionspuffer und eine Adsorptionsmatrix auf Basis von Kohlenhydraten zur Bindung von Verunreinigungen zusetzt und dann die Nukleinsäuren von der Adsorptionsmatrix und daran gebundenen Verunreinigungen abtrennt, wobei der Extraktionspuffer
(a) einen pH-Wert im Bereich von 3 bis 7,
(b) eine Salzkonzentration von mindestens 100 mM Alkalihalogenid oder/und
(c) eine phenolneutralisierende Substanz enthält.

Der Puffer weist einen pH-Wert im Bereich von 3 bis 7, vorzugweise von 4 bis 6,5 auf. Als günstig hat sich dabei die Verwendung von Acetatpuffern, z.B. Na-Acetat, erwiesen. Es können jedoch auch anderer Puffer, z.B. Phosphat- oder Citratpuffer, eingesetzt werden.

Weiterhin enthält der Extraktionspuffer eine Salzkonzentration von mindestens 100 mM Alkalihalogenid, vorzugsweise von mindestens 200 mM bis maximal zur Löslichkeitsgrenze des jeweils verwendeten Salzes. Als Salz wird ein Alkalihalogenid, z.B. NaCl oder KCI oder Gemische davon verwendet.

Weiterhin enthält der Puffer mindestens eine phenolneutralisierende Substanz. Bevorzugte Beispiele für Substanzen, die Phenole neutralisieren können, sind Polyvinylpyrrolidon (PVP) in unterschiedlichen Polymerisationsgraden, z.B. PVP-10, Reduktionsmittel, z.B. Thiolreagenzien wie β-Mercaptoethanol oder Dithiothreitol oder Borate. Besonders bevorzugt verwendet man Polyvinylpyrrolidon in einer Konzentration von mindestens 0,5% (Gew/Gew) bis zur Löslichkeitsgrenze.

Weiterhin enthalten die für das erfindungsgemäße Verfahren geeignete Extraktionspuffer vorzugsweise einen Chelatbildner wie etwa EDTA oder/und ein Detergenz, z.B. ein ionisches Detergenz wie SDS. Der Chelatbildner liegt vorzugsweise in einer Konzentration von 1 bis 200 mM vor. Die Konzentration des Detergenz beträgt vorzugsweise 0,1 bis 5% (Gew/Gew).

Die Adsorptionsmatrix ist derart beschaffen, daß sie in Verbindung mit dem Extraktionspuffer Verunreinigungen, die zur Schädigung von Nukleinsäuren führen oder/und die Durchführung enzymatischer Reaktionen verhindern oder/und enzymatische Reaktionen hemmen, wie etwa Abbauprodukte von Hämoglobin, z.B. Bilirubin und dessen Abbauprodukte, Gallensäuren oder Salze davon oder deren Abbauprodukte oder/und Polysaccharide und Polyphenole insbesondere pflanzlichen Ursprungs abtrennen oder neutralisieren kann. Die Verwendung einer unlöslichen Adsorptionsmatrix ist bevorzugt.

Bezüglich der geeigneten Adsorptionsmatrizes wird auf die Anmeldung PCT/EP96/03595 verwiesen. Es werden Adsorptionsmatrizes auf Basis von Kohlenhydraten verwendet, beispielsweise Mehle aus Getreide, Mais, Erbsen, Soja und insbesondere aus Kartoffeln oder Bestandteile daraus bzw. Mischungen davon. Besonders bevorzugt sind Mischungen von Mehlen mit anderen Kohlenhydraten, z.B. gereinigten Kohlenhydraten wie Cellulose.

Die Menge, in der die Adsorptionsmatrix der Probe zugesetzt wird, hängt im wesentlichen von der Beschaffenheit der Probe ab. Die Adsorptionsmatrix kann beispielsweise in einem Gewichtsanteil von 0,05:1 bis 100:1, insbesondere von 0,1:1 bis 10:1 bezüglich der Probe eingesetzt werden.

Die Nukleinsäuren enthaltende Probe stammt aus biologischen Materialien, die Nukleinsäure abbauende bzw. enzymatische Reaktionen hemmende Verunreinigungen enthalten. Vorzugsweise stammt die Probe aus Fäkalien. Sie kann jedoch auch beispielsweise aus anderen Quellen, z.B. Geweben jeder Art, Knochenmark, humanen und tierischen Körperflüssigkeiten wie Blut, Serum, Plasma, Urin, Sperma, Cerebrospinalflüssigkeit, Sputum und Abstrichen, Pflanzen, Pflanzenteilen- und extrakten, z.B. Säften, Pilzen, Mikroorganismen wie Bakterien, fossilen oder mumifizierten Proben, Bodenproben, Klärschlamm, Abwässern und Lebensmitteln stammen.

Vorzugsweise wird die Probe vor dem Zusatz der Adsorptionsmatrix im Extraktionspuffer aufgenommen und für eine jeweils gewünschte Zeitdauer vorinkubiert. Andererseits können Probe, Extraktionspuffer und Adsorptionsmatrix auch gleichzeitig zusammengegeben werden. Der Extrak-tionspuffer wird vorzugsweise in einem Gewichtsanteil von mindestens 0,1:1, insbesondere von 0,5:1 bis 50:1 bezüglich der Probe eingesetzt. Die Inkubation der Probe im Extraktionspuffer kann bei Raumtemperatur erfolgen und umfaßt vorzugsweise einen Homogenisierungsschritt z.B. durch Vortexbehandlung.

In einer Ausgestaltungsform der Erfindung kann die Inkubation unter Bedingungen erfolgen, die für eine Freisetzung der Nukleinsäuren aus dem Probenmaterial förderlich sind. Solche Inkubationsbedingungen werden insbesondere dann verwendet, wenn Nukleinsäuren aus "schwer" aufschließbaren Materialien, z.B. Zellen wie etwa Bakterien oder Parasiten oder Viren nachgewiesen werden sollen. In diesem Fall kann durch chemische, thermische oder/und enzymatische Behandlung die Freisetzung der Nukleinäsuren während der Inkubation verbessert werden, wodurch eine höhere Ausbeute an Nukleinsäuren aus dem Probenmaterial sowohl hinsichtlich Gesamt-DNA als auch spezifisch hinsichtlich der nachzuweisenden DNA erhalten wird. Vorzugsweise wird hierbei eine Temperaturerhöhung, z.B. auf ≥ 50°C, insbesondere auf ≥ 70°C vorgenommen.

Wenn andererseits Nukleinsäuren aus leicht aufschließbaren Materialien, z.B. empfindlichen Zellen wie etwa Humanzellen bestimmt werden sollen, kann die Inkubation auch bei einer verringerten Temperatur, z.B. ≤ 10°C, insbesondere ≤ 4°C erfolgen, um auf diese Weise die unerwünschte Freisetzung anderer Nukleinsäuren in der Probe zu vermeiden oder einzuschränken.

Nach Zusatz der Adsorptionsmatrix erfolgt eine weitere Inkubation der Probe. Auch diese Inkubation kann - je nach Erfordernis - bei Raumtemperatur, bei einer verringerten Temperatur oder bei für die Freisetzung von Nukleinsäuren förderlichen Bedingungen erfolgen.

Nach der Inkubation kann die Adsorptionsmatrix z.B. durch Zentrifugation von der Probe abgetrennt werden. Alternativ kann die Probe direkt mit der Adsorptionsmatrix versetzt werden, z.B. bei flüssigen biologischen Proben. Weiterhin kann die Probe über eine Adsorptionsmatrix durch Zentrifugation, durch Anlegen eines Vakuums oder/und mittels der Schwerkraft geführt werden, wobei die Adsorptionsmatrix vorzugsweise dann abgefüllt in einer Säule vorliegt.

Die Behandlung mit Extraktionspuffer und Adsorptionsmatrix führt zu einer signifikanten Stabilitätserhöhung der in der Probe enthaltenen Nukleinsäuren und bei einer anschließenden Isolierung der Nukleinsäuren zu einer besseren Reproduzierbarkeit. Dies gilt insbesondere, wenn sich an die Isolierung eine enzymatische Manipulation der Nukleinsäuren, z.B. eine Amplifikation oder/und eine Restriktionsspaltung anschließt. Besonders bevorzugt wird die Amplifikation, z.B. durch PCR (Polymerase Chain Reaction), LCR (Ligase Chain Reaction), NASBA (Nucleic Acid Base Specific Amplification) oder 3SR (Self Sustained Sequence Replication) durchgeführt.

Ein besonders bevorzugter Aspekt der vorliegenden Erfindung ist - wie bereits in PCT/EP96/03595 ausgeführt - die Analyse, der Nachweis oder die Isolierung von Nukleinsäuren, insbesondere DNA, aus Stuhlproben. Durch das erfindungsgemäße Verfahren sind saubere und amplifizierbare Nukleinsäuren aus fäkalen Proben erhältlich, die zum Nachweis von Mutationen, insbesondere von tumorspezifischen DNA-Mutationen verwendet werden können.

Ein weiterer Gegenstand der vorliegenden Erfindung ist ein Reagenzienkit zur Stabilisierung und Reinigung von Nukleinsäuren aus biologischen Materialien, umfassend:
(a) einen zur Aufnahme einer Nukleinsäuren enthaltenden Probe geeigneten Extraktionspuffer wie zuvor beschrieben,
(b) eine Adsorptionsmatrix auf Basis von Kohlenhydraten zur Bindung von Verunreinigungen aus den biologischen Materialien.

Die Adsorptionsmatrix kann in einer abgepackten portionierten Form, z.B. abgefüllt in einer Säule wie etwa einer zentrifugierbaren Minisäule, vorliegen. Der Puffer kann in fertiger Form, als Konzentrat oder Lyophilisat vorliegen.

Vorzugsweise enthält das Reagenzienkit zusätzliche Mittel zur Reinigung von Nukleinsäuren, die z.B. mineralische oder/und organische Träger sowie gegebenenfalls Lösungen, Hilfsstoffe oder/und Zubehör umfassen. Mineralische Bestandteile von Trägern können beispielsweise poröse oder nicht-poröse Metalloxide oder Metallmischoxide, z.B. Aluminiumoxid, Titandioxid oder Zirkoniumdioxid, Silicagele, Materialien auf Basis von Gläsern, z. B. modifizierte oder nicht-modifizierte Glaspartikel oder Glasmehl, Quarz, Zeolithe oder Mischungen von einer oder mehrerer der oben genannten Substanzen sein. Andererseits kann der Träger auch organische Bestandteile enthalten, die z.B. aus gegebenenfalls mit funktionellen Gruppen modifizierten Latexpartikein, synthetischen Polymeren, wie etwa Polyethylen, Polypropylen, Polyvinylidenfluorid, insbesondere ultrahochmolekularem Polyethylen oder HD-Polyethylen, oder Mischungen von einer oder mehreren der zuvor genannten Substanzen ausgewählt werden.

Der Träger kann beispielsweise in Form von Partikeln mit einer mittleren Größe von 0,1 µm bis 100 µm vorliegen. Bei Verwendung eines porösen Trägers ist eine mittlere Porengröße von 2 µm bis 100 µm bevorzugt. Der Träger kann beispielsweise in Form loser Schüttungen von Partikeln, Filterschichten, z.B. aus Glas, Quarz oder Keramik, Membranen, z.B. Membranen, in denen ein Silicagel angeordnet ist, Fasern oder Geweben aus mineralischen Trägern, wie etwa Quarz oder Glaswolle sowie in Form von Latices oder Frittenmaterialien aus synthetischen Polymeren vorliegen.

Außerdem kann das erfindungsgemäß Reagenzienkit auch Hilfsstoffe wie Enzyme und andere Mittel zur Manipulation von Nukleinsäuren enthalten, z.B. mindestens einen Amplifikationsprimer und zur Amplifikation von Nukleinsäuren geeignete Enzyme, z.B. eine Nukleinsäurepolymerase oder/und mindestens eine Restriktionsendonuklease.

Die Primer zur Amplifkation von Nukleinsäuren stammen zweckmäßigerweise aus den zu analysierenden Genen, dh. beispielsweise aus Onkogenen, Tumorsupressorgenen oder/und Mikrosatellitenabschnitten. zur Amplifikation von Nukleinsäuren geeignete Enzyme und Restriktionsendonukleasen sind bekannt und kommerziell erhältlich.

Weiterhin soll die vorliegende Erfindung durch die nachfolgenden Abbildungen und Beispiele erläutert werden. Es zeigt:

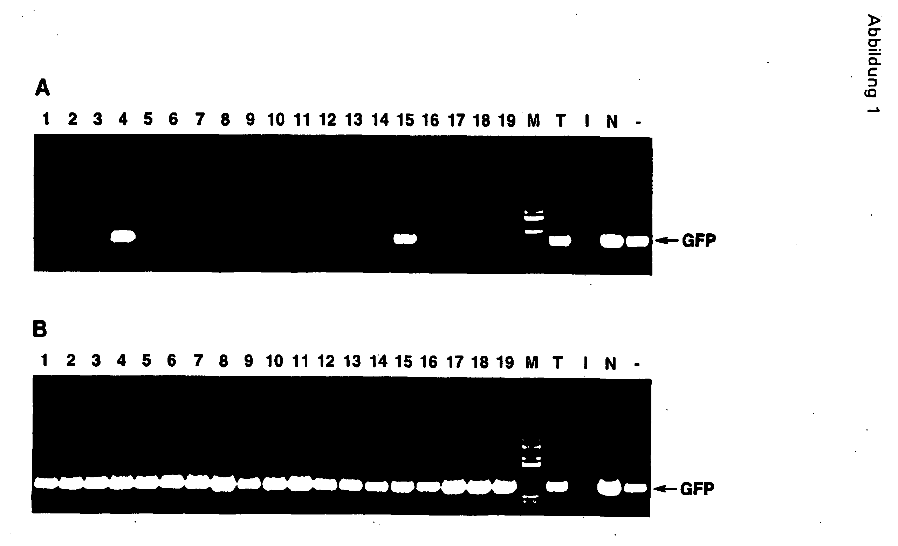

- Abb 1.:: die Amplifizierbarkeit von DNA in inhibitorischen Stuhlproben unter Verwendung eines Extraktionspuffers des Standes der Technik (Abb. 1a) und eines erfindungsgemäßen Extraktionspuffers (Abb. 1 b).

### Beispiel 1

### Analyse von DNA aus Stuhlproben

DNA wurde aus Stuhlproben unter Verwendung einer Adsorptionsmatrix aus Cellulose und Kartoffelmehl gereinigt und anschließend mittels PCR amplifiziert.

Menschliche Stuhlproben wurden gesammelt, eingefroren und bei -80°C aufbewahrt. 200 mg Stuhl wurden in ein 2 ml Mikrozentrifugengefäß gegeben und auf Eis gestellt. Dann wurde die Stuhlprobe in 600 µl Extraktionspuffer aufgenommen und die Mischung durch Vortexbehandlung für 1 min homogenisiert.

Die auf Kartoffelmehl und Cellulose basierende Adsorptionsmatrix (200 mg) wurde in 300 µl Extraktionspuffer aufgenommen und durch Vortexbehandlung resuspendiert. Dann wurde die Matrix-Suspension dem Stuhl-Homogenisat zugesetzt und einer Vortexbehandlung für 1 min unterzogen.

Die Probe wurde für 5 min zur Präzipitation von Stuhlpartikeln, der Adsorptionsmatrix und anderen Verunreinigungen zentrifugiert. Der Überstand wurde in ein neues Mikrozentrifugationsgefäß überführt und für weitere 5 min zentrifugiert.

Die in 600 µl des Überstands enthaltene DNA wurde mit Hilfe von Reagenzien und Zentrifugationssäulen wie folgt noch weiter aufgereinigt. Nach einer Proteinase K-Behandlung wurden die Nukleinsäuren in Gegenwart chaotroper Salze an einer Silikagelmembran einer Zentrifugationssäule gebunden und nach wiederholten Waschschritten eluiert.

Anschließend wurde den DNA-Eluaten eine Matrize (eine für GFP (Green Fluorescence Protein) kodierende DNA) sowie die weiteren für dessen Amplifikation notwendigen Komponenten (Primer, Polymerase, Nukleotide, Puffer) zugesetzt. Die Endkonzentration der DNA-Eluate im PCR-Ansatz betrug 10% (Vol/Vol).

Es wurden DNA-Isolate aus inhibitorischen Stuhlproben von insgesamt 19 Personen mittels PCR auf Amplifizierbarkeit getestet (Spuren 1 bis 19 in Abb. 1a und b). Die Ansätze wurden nach der PCR durch Gelelektrophorese aufgetrennt und die Amplifikationsprodukte (zu erwartende Länge 771 bp) durch Ethidiumbromidfärbung visualisiert.

Als Referenz wurde ein DNA Längenmarker (M; 1 kB-Marker, Gibco BRL, Bethesda Maryland) auf das Gel aufgetragen. Als Kontrolle wurden dem GFP-PCR-Ansatz anstelle der DNA-Eluate Tris-Puffer (T), eine stark inhibitorische Stuhl-DNA (I), oder eine nicht inhibitorische Stuhl-DNA (N) zugefügt. Desweiteren wurde in einer Kontrollreaktion GFP ohne jegliche Zusätze (-) amplifiziert.

Bei inhibitorischen Stuhlproben konnte unter Verwendung des in PCT/EP96/03595 verwendeten Stuhl-Lösepuffers (500 mM Tris HCl pH 9,0, 50 mM EDTA, 10 mM NaCl) oftmals kein Amplifikationsprodukt erhalten werden. So ist aus Abb. 1 a ersichtlich, daß mit dem aus PCT/EP96/03595 bekannten Protokoll nur bei zwei von 19 getesteten Proben (Proben Nr. 4 und 15) eine Amplifikation erfolgte.

Überraschenderweise wurde festgestellt, daß die Amplifizierbarkeit der DNA durch Ersetzen des Standardpuffers mit einem in der folgenden Tabelle 1 gezeigten Puffer E1 bis E8 dramatisch verbessert werden konnte.

**Tabelle 1**

| | **NaAcetat** | **NaCl** | **KCI** | **EDTA** | **SDS** | **PVP-10** | **pH** |
|---|---|---|---|---|---|---|---|
| E1 | 0,2 M | 2,5 M | - | 60 mM | 1,5% | 2% | 6,5 |
| E2 | 0,2 M | 0,5 M | - | 50 mM | 1,4% | 3% | 5,0 |
| E3 | 0,1 M | 1,0 M | - | 60 mM | 1,0% | 4% | 6,0 |
| E4 | 0,1 M | 0,5 M | - | 50 mM | 1,4% | 2% | 5,5 |
| E5 | 0,3 M | - | 0,1 M | 80 mM | 1,5% | 3% | 6,0 |
| E6 | 0,1 M | - | 0,2 M | 50 mM | 1,4% | 2% | 5,5 |
| E7 | 0,3 M | - | 0,5 M | 60 mM | 1,0% | 1% | 4,0 |
| E8 | 0,2 M | - | 0,1 M | 60 mM | 1,0% | 1% | 6,5 |

Abbildung 1b zeigt, daß bei Verwendung eines erfindungsgemäßen Extraktionspuffers aus allen 19 Proben eine amplifizierbare DNA isoliert werden konnte.

### Beispiel 2

### Stuhlextraktion bei erhöhter Temperatur

Für die Detektion von Nukleinsäuren aus bestimmten Zellen (z.B. Bakterien, Parasiten) oder Viren ist eine Extraktion der Stuhlprobe bei erhöhten Temperaturen zweckmäßig, um eine effiziente Freisetzung der DNA zu gewährleisten.

Jeweils 1 g Stuhl wurde mit 10⁵ Agrobakterien versetzt und entsprechend der Methode in Beispiel 1 aufgearbeitet. Die Extraktion der Stuhlprobe in einem erfindungsgemäßen Puffer erfolgte dabei für 5 min bei 4°C, Raumtemperatur von 18-25°C (RT), 50°C, 70°C, 80°C oder 90°C. Die Effizienz der Lyse wurde über die Gesamt-DNA Ausbeute und die Effizienz der Lyse der zugesetzten Agrobakterien über die Amplifikation einer spezifischen Agrobakteriensequenz (Vir-Gen) bestimmt. Die Ergebnisse sind in der folgenden Tabelle 2 gezeigt.

**Tabelle 2**

| **Temperatur** | **Gesamt-DNA Ausbeute (ng/**µ**l)** | **Vir Amplifikation** |
|---|---|---|
| 4°C | 115 | + |
| RT | 161 | + + |
| 50°C | 255 | + + + |
| 70°C | 536 | + + + + |
| 80°C | 521 | + + + + |
| 90°C | 548 | + + + + |

Den Ergebnissen liegen jeweils zwei unabhängige Stuhlextraktionen bei der angegebenen Temperatur zugrunde. Die Gesamt- DNA Ausbeute wurde über OD-Messung bei 260 nm bestimmt. Die Amplifikationsprodukte wurden auf einem Agarosegel aufgetrennt. Mit + ist die Effizienz der Amplifikation bezeichnet (+ bis + + + + : zunehmende Effizienz).

Aus Tabelle 2 ist ersichtlich, daß sowohl die Gesamt-DNA Ausbeute als auch die Bakterienlyse und somit die Amplifikationsausbeute bei einer Erhöhung der Inkubationstemperatur auf mindestens 50°C, insbesondere auf mindestens 70°C deutlich zunimmt.

## Patentansprüche

1. Verfahren zur Reinigung, Stabilisierung oder/und Isolierung von Nukleinsäuren aus biologischen Materialien, wobei man der Nukleinsäuren enthaltenden Probe einen Extraktionspuffer und eine Adsorptionsmatrix auf Basis von Kohlenhydraten zur Bindung von Verunreinigungen zusetzt und dann die Nukleinsäuren von der Adsorptionsmatrix abtrennt,
**dadurch gekennzeichnet,**
**dass** der Extraktionspuffer
(a) einen pH-Wert im Bereich von 3-7,
(b) eine Salzkonzentration von mindestens 100 mM Alkalihalogenid und
(c) eine phenolneutralisierende Substanz
enthält.

2. Verfahren nach Anspruch 1,
**dadurch gekennzeichnet,**
**dass** man einen Extraktionspuffer mit einem pH-Wert von 4-6,5 verwendet.

3. Verfahren nach Anspruch 1 oder 2,
**dadurch gekennzeichnet,**
**dass** man einen Extraktionspuffer mit KCI oder/und NaCl in einer Konzentration von mindestens 100 mM verwendet.

4. Verfahren nach einem der vorhergehenden Ansprüche,
**dadurch gekennzeichnet,**
**dass** man einen Extraktionspuffer mit mindestens 0,5 % Polyvinylpyrrolidon als phenolneutralisierende Substanz verwendet.

5. Verfahren nach einem der vorhergehenden Ansprüche,
**dadurch gekennzeichnet,**
**dass** man eine unlösliche Adsorptionsmatrix auf Kohlenhydratbasis verwendet.

6. Verfahren nach einem der vorhergehenden Ansprüche,
**dadurch gekennzeichnet,**
**dass** man Kartoffelmehl oder Bestandteile daraus gegebenenfalls vermischt mit anderen Kohlenhydraten verwendet.

7. Verfahren nach einem der vorhergehenden Ansprüche,
**dadurch gekennzeichnet,**
**dass** die Nukleinsäuren enthaltende Probe aus Fäkalien stammt.

8. Verfahren nach einem der vorhergehenden Ansprüche,
**dadurch gekennzeichnet,**
**dass** die Probe vor dem Inkontaktbringen mit der Adsorptionsmatrix im Extraktionspuffer inkubiert wird.

9. Verfahren nach Anspruch 8,
**dadurch gekennzeichnet,**
**dass** die Inkubation bei einer Temperatur ≤ 10 °C erfolgt.

10. Verfahren nach Anspruch 8,
**dadurch gekennzeichnet,**
**dass** die Inkubation bei Bedingungen erfolgt, die für eine Freisetzung der Nukleinsäuren förderlich sind, umfassend eine chemische, thermische oder/und enzymatische Behandlung.

11. Verfahren nach Anspruch 10,
**dadurch gekennzeichnet,**
**dass** die Inkubation bei einer Temperatur ≥ 50 °C erfolgt.

12. Verfahren nach einem der vorhergehenden Ansprüche,
**dadurch gekennzeichnet,**
**dass** man die Probe durch Zentrifugation, durch Anlegen eines Vakuums oder/und mittels der Schwerkraft über die Adsorptionsmatrix führt.

13. Verwendung eines Verfahrens nach einem der Ansprüche 1 bis 12 zur Analyse, zum Nachweis oder zur Isolierung von Nukleinsäuren aus Stuhlproben.

14. Reagenzienkit zur Reinigung, Stabilisierung oder/und Isolierung von Nukleinsäuren aus biologischen Materialien umfassend:
(a) einen zur Aufnahme einer Nukleinsäure enthaltenden Probe geeigneten Extraktionspuffer wie in einem der Ansprüche 1 bis 4 definiert und
(b) eine Adsorptionsmatrix auf Basis von Kohlenhydraten zur Bindung von Verunreinigungen aus den biologischen Materialien.

## Claims

1. Method for purifying, stabilizing or/and isolating nucleic acids from biological materials in which an extraction buffer and an adsorption matrix based on carbohydrates are added to the sample containing nucleic acids in order to bind impurities and then the nucleic acids are separated from the adsorption matrix,
**characterized in that**
the extraction buffer
(a) has a pH in the range of 3 - 7,
(b) has a salt concentration of at least 100 mM alkali halogenide and
(c) contains a phenol-neutralizing substance.

2. Method according to claim 1,
**characterized in that**
an extraction buffer with a pH of 4 - 6.5 is used.

3. Method according to claim 1 or 2,
**characterized in that**
an extraction buffer containing KCl or/and NaCl at a concentration of at least 100 mM is used.

4. Method according to one of the previous claims,
**characterized in that**
an extraction buffer containing at least 0.5 % polyvinyl pyrrolidone as the phenol-neutralizing substance is used.

5. Method according to one of the previous claims,
**characterized in that**
an insoluble adsorption matrix based on carbohydrates is used.

6. Method according to one of the previous claims,
**characterized in that**
potato flour or components thereof optionally mixed with other carbohydrates is used.

7. Method according to one of the previous claims,
**characterized in that**
the sample containing nucleic acids originates from faeces.

8. Method according to one of the previous claims,
**characterized in that**
the sample is incubated in the extraction buffer before being contacted with the adsorption matrix.

9. Method according to claim 8,
**characterized in that**
the incubation is carried out at a temperature of ≤ 10°C.

10. Method according to claim 8,
**characterized in that**
the incubation takes place under conditions which are beneficial for a release of nucleic acids, comprising a chemical, thermal or/and enzymatic treatment.

11. Method according to claim 10,
**characterized in that**
the incubation takes place at a temperature of ≥ 50°C.

12. Method according to one of the previous claims,
**characterized in that**
the sample is conveyed over the adsorption matrix by centrifugation, by applying a vacuum or/and by means of gravitational force.

13. Use of a method according to one of the claims 1 to 12 for analysing, detecting or isolating nucleic acids from stool samples.

14. Reagent kit for purifying, stabilizing or/and isolating nucleic acids from biological materials comprising:
(a) an extraction buffer as defined in one of the claims 1 to 4 suitable for taking up a sample containing nucleic acid and
(b) an adsorption matrix based on carbohydrates for binding impurities from the biological materials.

## Revendications

1. Procédé pour purifier, stabiliser et/ou isoler des acides nucléiques à partir de matières biologiques où l'on ajoute à l'échantillon contenant des acides nucléiques un tampon d'extraction et une matrice d'adsorption à base de glucides pour la liaison des impuretés puis on sépare les acides nucléiques de la matrice d'adsorption, **caractérisé en ce que** le tampon d'extraction a
(a) un pH dans la plage de 3-7,
(b) une concentration de sels d'au moins 100 mM d'halogénure alcalin et
(c) une substance neutralisant les phénols.

2. Procédé selon la revendication 1 **caractérisé en ce que** l'on utilise un tampon d'extraction d'un pH de 4-6,5.

3. Procédé selon la revendication 1 ou 2 **caractérisé en ce que** l'on utilise un tampon d'extraction avec KCl et/ou NaCl en une concentration d'au moins 100 mM.

4. Procédé selon l'une des revendications précédentes **caractérisé en ce que** l'on utilise un tampon d'extraction avec au moins 0,5 % de polyvinylpyrrolidone comme substance neutralisant les phénols.

5. Procédé selon l'une des revendications précédentes **caractérisé en ce que** l'on utilise une matrice d'adsorption insoluble à base de glucides.

6. Procédé selon l'une des revendications précédentes **caractérisé en ce que** l'on utilise de la farine de pommes de terre ou des constituants de celle-ci éventuellement en mélange avec d'autres glucides.

7. Procédé selon l'une des revendications précédentes **caractérisé en ce que** l'échantillon contenant des acides nucléiques est issu de matières fécales.

8. Procédé selon l'une des revendications précédentes **caractérisé en ce que** l'échantillon est incubé dans le tampon d'extraction avant la mise en contact avec la matrice d'adsorption.

9. Procédé selon la revendication 8 **caractérisé en ce que** l'incubation a lieu à une température ≤10°C.

10. Procédé selon la revendication 8 **caractérisé en ce que** l'incubation a lieu dans des conditions qui sont utiles pour une libération des acides nucléiques, comprenant un traitement chimique, thermique et/ou enzymatique.

11. Procédé selon la revendication 10 **caractérisé en ce que** l'incubation a lieu à une température ≥50°C.

12. Procédé selon l'une des revendications précédentes **caractérisé en ce que** l'on conduit l'échantillon sur la matrice d'adsorption par centrifugation, par application d'un vide et/ou au moyen de la force de pesanteur.

13. Utilisation d'un procédé selon l'une des revendications 1 à 12 pour l'analyse, pour la mise en évidence ou pour l'isolement d'acides nucléiques à partir d'échantillons de selles.

14. Kit de réactifs pour purifier, stabiliser et/ou isoler des acides nucléiques à partir de matières biologiques comprenant :
(a) un tampon d'extraction approprié pour recevoir un échantillon contenant des acides nucléiques tel que défini dans l'une des revendications 1 à 4 et
(b) une matrice d'adsorption à base de glucides pour la liaison des impuretés issues des matières biologiques.
